# EUROPEAN PATENT SPECIFICATION

(11) **EP 1 902 184 B1**
(45) Date of publication and mention of the grant of the patent: **13.04.2016**
(21) Application number: 06788006.2
(22) Date of filing: 20.07.2006
(51) Int. Cl.: A47L 11/282, A47L 11/40, A61B 50/13, A61B 50/36, B01L 1/02, B65F 1/06, E04B 5/48

(54) **HOSPITAL OPERATING ROOM RE-DESIGN**
NEUGESTALTUNG EINES OP-RAUMS
NOUVELLE CONCEPTION DE SALLE D'OPERATION D'HOPITAL

(30) Priority: 20.07.2005 US 701106 P
(43) Date of publication of application: 26.03.2008
(73) Proprietor: Optimus Licensing AG, 6300 Zug (CH)
(72) Inventor: MANGIARDI, John, R., Greenwich, CT 06830 (US)
(74) Representative: Manitz, Finsterwald & Partner GbR
(86) International application number: PCT/US2006/028226
(87) International publication number: WO 2007/012040

(56) References cited:
- DE-U1- 9 217 542
- FR-A1- 2 699 747
- US-A- 5 097 950
- US-A- 5 959 423
- US-A- 5 967 780
- US-A- 5 992 108
- US-A1- 2002 108 177
- US-A1- 2003 161 152
- US-A1- 2003 224 729
- US-A1- 2003 224 729
- US-A1- 2005 133 234
- US-B1- 6 202 360
- US-B1- 6 202 360
- US-B1- 6 311 441
- US-B1- 6 415 552

## Description

The present invention relates to an operating room in accordance with the preamble of claim 1 and comprising walls, a ceiling;
a sub-floor and a finished floor as well as to a method of using an operating room to maximize space and/or increase safety in accordance with the introduction to claim 18.

Thus the present invention is aimed at providing more efficient operating room configurations and controls. In particular, the present invention relates to an operating room configuration that among other things: increases and maximizes operating room space, provides a more sterile operating room, allows more efficient cleaning, eliminates apparatus wires, hoses, and cables, and provides data integration and a sense of calm for patients and staff.

An operating room of the initially named kind is known from US-A-6,202,360. There a computer and an anesthia center are shown positioned between the sub-floor and the finisked floor and the connections to them are provided above the finished floor.

### BACKGROUND OF THE INVENTION

The operating room and the preoperative process are at the core of hospital care. The operating room is a high-cost and high-risk environment. It must accommodate multiple surgical specialties with attendant specialized technologies and provide care for an inordinate number of differing, unique cases. As a result, the operating room is the most difficult environment to manage in healthcare. While surgical techniques, clinical devices, and the development of technological advances have pushed operative medicine into the future, operating room informatics have lagged behind. Surgeons are surrounded by sophisticated clinical equipment to help them operate on or monitor their patient, but often cannot get basic information about their cases. Operating room environments frequently lead to inefficiency, poorly coordinated scheduling, long room-turnover times, poor communication amongst team members, medical errors, and user dissatisfaction. The current operating room environment is simply not conducive to the universal hospital goals of decreasing costs, increasing quality and incremental volume, and market share. Attempts have been made to address particular problems. For example, an attempt to manage the overcrowding of operating room floors has been made by the design decision to place utility connections on the ceiling. Yet solutions such as this, without an eye to the holistic design of the operating room, create as many problems as they attempt to fix. As in the former example, while wires and cabling are removed from the floor, they now dangle from the ceiling again creating a hazard.

Of the many unresolved problems associated with operating room environments, some of the most critical can be summarized as follows:
1) Lack of Focus: The operating room is structured without any specific area of focus, and is therefore, by design, more complicated than simple. This is a result of principles followed from the time of World War II when operating rooms were designed to accommodate multiple operations in a single room. There is duplication of infrastructure throughout the room that is no longer necessary as only one patient undergoes an operation at one time in the modem non-military operating room.
2) Operating Table Lacks Safety: The operating table is not fixed in place and under certain circumstances can become unstable or may move accidentally during surgery. Operating tables have little or no parallel functionality other than to position a patient. A general operating room requires between two to three operating tables per room to support different surgical specialties, resulting in crowding and increased overhead cost.
3) Cluttered/Lack of Safety & Sterility: Clutter is one of the main challenges to patient safety in the operating room environment, as individual technological developments have resulted in a dramatic increase in the number and complexity of operating room equipment and supplies. Heavy equipment, some of which is permanently installed and some of which is moved in and out of the operating room during an operation, creates obstacles to movement of staff and equipment. The floor is covered with electrical wiring, vacuum tubes, gas hoses, rolling and fixed garbage receptacles. These represent serious safety hazards to nurses, technicians, physicians and patients. Intra-operative complications due to staff injury and equipment dislocation have caused dramatic clinical consequences for patients and liability for both physicians and hospitals.
4) Inefficient Use of Walls/Lack of Sterility: Operating room walls are not multifunctional and are used only to mount X-ray viewing boxes and wall outlets. The operating room walls are made of painted plasterboard that limits sterility. The wall material has a high dielectric constant and therefore gathers and holds onto dust and aerosols, infected or otherwise, for long periods of time. The walls cannot be scrubbed down, as most paint materials do not tolerate high friction when surfaced on plasterboard. Some hospitals have tile walls that can be scrubbed; however, the grouting materials do not release grime and pyrogens. Current practice is that operating room walls are rarely scrubbed down, and are, therefore, not sterile. Operating room cabinets are too small and have limited capacity for par stocking of supplies. Insufficient par stocking results in prolonged turnover time.
5) Inefficient Lighting/Lack of Sterility & Safety: Ceiling-mounted surgical lights are cumbersome and arcane. They also represent a safety hazard to the surgical staff. They poorly illuminate the surgical field, are difficult to maneuver, and compete for precious space needed for other intra-operative technology. They are difficult to keep clean and sterilize and are a source of breaks in sterile technique and of falling microbe-laden dust.
6) Contained Floors/Lack of Sterility: Floors are typically washed down between cases with a bucket and re-used mops (or with disposable swipes). These materials are used from room to room, and from case to case. Unfortunately, the floors are not sterile, and infection can be transmitted from room to room. To date, no reliable and simplified mechanism for sterilizing an operating room floor is available.
7) Inefficient Re-stocking/Lack of Safety: The current practice of re-stocking the operating room with required supplies and equipment during surgery relies on the availability of a circulating nurse. The process is not only inefficient, but it is also dangerous, as the surgeon must wait for the required supplies when needed. If a complication occurs or worsens while the circulating nurse is out of the room searching for needed supplies, the surgeon and staff are left with no one to obtain necessary items in an urgent situation.
8) Lack of Space: In general, the modem operating room is too small (typically 300 to 550 square feet), and not capable of handling the proliferation of advanced technology that has been introduced over the past decade.
9) Lack of Real-Time Information: The lack of real-time information accession has resulted in an environment that is inefficient, leading to lost time and hospital errors when critical data is missing. Information sharing of patient information, radiology studies, test results, and pathology reports is limited. Despite the technological digital revolution that has taken place in our offices and our homes, operating room information technology has progressed little since the 1970's.
10) Lengthy Turnover Time: In addition to the inefficient re-stocking, the room design contributes little or nothing to a reduction in turnover time, resulting in cost overruns both in terms of personnel and overtime costs.
11) Lack of Sense of Calm and Comfort: For the patient, the operating room is not a place that inspires a sense of calm, confidence, and trust. Patient satisfaction is impacted by the garish environment. The most common fearful experience that surgical patients relate when queried is that of their entry into the operating room. The ergonomic character of the room is extremely limited, resulting in limited comfort, line-of-sight, and sense of calm for the surgeon and operating room staff. Further, patient fear contributes to increased time needed to sedate the patient with a further concomitant increase in turn-over time.

It is an object of the present invention to provide an efficiently configured and controlled surgical operating room/health care treatment room/suite that resolves one or more of the above limitations of the current design. In addition, additional objects will become apparent after consideration of the following descriptions and claims.

### SUMMARY OF THE INVENTION

In order to satisfy the above object there is provided, in accordance with the invention, an operating room in accordance with claim 1 and a method in accordance with claim 18.

More specifically, in keeping with these objects and others that may become apparent, the present invention comprises a number of design changes and incorporates improved operating room apparatuses.

### Floor Pods

A number of centrally located "floor pods" containing utility connections are provided. A utility comprises electrical, gas, vacuum, water, data-line, and other support connections. Each "floor pod" is retracted back into the floor when not in use and is adapted to provide a UL electrical, fire, and water rated flooring. The floor provides "below-floor" connections for the pods and other utilities.

Each "floor pod" can be connected directly to a surgical cart, such as an anesthesia cart, an endoscopy cart, or a laparoscopy cart, providing alternative available locations to meet the space requirements of the specific procedure. Carts are designed to present connecting outlets for utilities mounted on a newly designed receiving grid located on the bottom of the cart, providing more space for patient monitoring needs such as by an anesthesiologist, endoscopist, or laparoscopist.

Existing power/gas columns/pods are removed from the ceiling and replaced by floor pods in the floor. Pods are raised when needed and are retractable into the floor when not in use. This relocation both frees up ceiling space (for relocated imaging) and eliminates cords and connecting wires hanging down and intruding into critical surgical space.

### Wireless Integration

The operating room features wireless transmission of data to the extent possible, with remaining necessary cable connections located underneath the floor. This removes cables and hoses from the floor for a safer, easier to clean environment. Most current-generation electronic equipment comes equipped with wireless capabilities or can be made wireless.

Further, wireless universal serial bus (USB) to radio frequency (RF) to universal serial bus (USB) paired input and output devices wirelessly connect "in-room" equipment, e.g., microscopes, diagnostic imaging equipment, anesthesia monitoring, navigational devices, endoscopes, etc., to wall-mounted high-definition display monitors.

Data management will be integrated into a software system known as "GCQ," currently in use at UCLA. This data management system manages data from the hospital information system, laboratory data system, radiology ("PACS"), and local input by the nursing staff during the life of an operation. The system includes input from wireless USB data ports from such technology introduced into the operating room as the operating microscope, ultrasound, laparoscopy tower, navigational computer system, anesthesia machine, C-arm radiology, etc.

For instance, in one embodiment, a surgical microscope is brought into the room and is being used by the surgeon. The microscope has a USB video output slot. A USB transmitter is inserted into that slot, and a paired USB receiver is inserted into an available display USB slot. The GCQ software discovers a new device and displays the appropriate icon on the screen. Once the icon is activated (as by a laser touch screen, discussed below), the video image is presented on the display panel within one of the GCQ windows.

### High-Definition Displays

In a preferred embodiment, at least three large-screen high-definition monitors are mounted on surrounding walls, thereby providing imaging and patient information in real time and overall informatic integration. Further, the monitors provide excellent viewability by anyone from anywhere in the room as opposed to current displays which are integrated onto individual operating room apparatuses. These displays may allow access to information via laser touch controls. Each monitor has "smart-panel" design capabilities allowing the physician to display information on any monitor within the suite. For example, dynamic data management within the room and with ancillary services (radiology IPACS, pathology, PACU, anesthesia, and hospital HIS) are displayed in real time in partitioned areas of the surrounding visual displays.

### Improved Surgical Tables

The patient tables are modified with a fixed-base design thereby allowing for heavier patients, greater tilting and cantilevering capabilities, greater table top extension (i.e. providing head to toe coverage), and overall stability. The operating room re-design includes incorporation of a "pod" concept into the architecture of the table, so that utilities, such as wiring, vacuum, and gas hoses, arise from the table itself, rather than tracking over the operating room floor. The source of all utilities to the table (as opposed to those emanating from the table) may come from an underground connection to a below floor source.

### Post Office Style Wall

A typical operating room can be enlarged by removal of operating room sinks and benchtops, thereby resulting in an increase in size. Items typically placed on or near the circulating hallway or even on the operating room floor can be relocated to storage cabinets in the operating room, thereby resulting in further clearance of hallways and reclamation of space.

The supplied storage cabinets are built into one or more walls of the operating room and are sufficient in size to allow stocking with all supplies and equipment required for the day's cases. This reduces the necessity of operating room staff having to leave the operating room to get additional supplies or equipment during cases. A "Post Office" style compartmentalization wall is used for easy access to stocked surgical supplies.

For example, in one embodiment, one wall in the suite includes floor to ceiling "Post Office" style compartments. The interior and exterior wall is adapted to provide glass doors on both sides. As such, an outside circulating nurse may open an exterior wall door, place any required supplies, and close the exterior wall door. A surgeon may then readily locate the supply because of the transparent nature of glass doors. After location, he or she may open the door, retrieve the needed supplies, and close the door. At any point in this process, there is no open area to both the operating room and the outside room at the same time, which thereby aids in maintaining sterility.

All supplies can be stocked from the outside of the operating room. All supplies can be coded (e.g., bar coded or radio frequency identification tagged) for easy on-site inventory control. All supplies removed from the shelves from the inside of the suite are easily coded for billing and inventory control. Surgical packs can be prepared the night before the procedure and identified for the physician/case. The "Post Office" style thereby eliminates the need for the circulating nurse to leave the suite during the case to retrieve supplies.

Additionally, the radio-frequency identification tagged ("RFID") devices can be particularly adapted for use in the operating room. It is contemplated that all items to be stocked on a shelf, such as (and preferably) a shelf in the post-office style wall, will be tagged. An antenna/locator/detector device on the shelf will determine which tagged items have been placed on the shelf and communicate with a central computer and database. The communication will log stocking and removal of items for inventory control. Further, items in which sterility affects usability can be tagged with a breakable RFID marker, such that when the item is opened, the RFID tag no longer communicates with the antenna/locator device. As such, the item is automatically removed from the inventory, again for inventory control.

### Imaging Devices

The traditional large, cumbersome radiology equipment, including a floor-mounted C-arm, image intensifier and supporting electronics is replaced by a new design with increased capabilities. A ceiling-mounted, miniaturized (thus lightened) C-arm with flat panel image intensifier and single-slice CT capability is provided. This is required to visualize and locate all implanted devices immediately after implantation before the patient is removed from the operating room. Imaging is one aspect of the redesign; in addition to producing improved imaging capabilities over the current system, it frees up considerable floor space, improves access to the operating table, and eliminates cabling on the floor.

In a typical embodiment, the imaging device comprises robotic technology utilizing air-gears and an ultra-light, fully computerized, digitized, and motorized imaging arm (C-arm). The arm uses a high-frequency generator and high-resolution imaging receptors. Finally, the arm is installed to a single-point in the ceiling, the point mounting eliminating the mounting tracks currently used in most facilities. The imaging device can also easily be tilted away when not in use.

A new C-arm unit can be made from improvements to existing imaging equipment, such as those manufactured by Siemens Medical Systems.

### Operating Room Ambient Lighting

The walls of the operating room can be fashioned out of a material that allows them to be backlit, thus allowing the room to be lit from all sides in varying intensities. Since the color of the back lighting can be changed, it produces a mood-enhancing environment that can lessen the anxiety level of a patient and provide a comfortable working environment for surgeons and staff. Further, the backlit material is preferably non-porous and of a low dielectric constant. Such a material is more sterile and is capable of being cleaned. Such backlit wall lighting is commercially available. For example, Avonite® wall covering may be used. Avonite® is a translucent material that attaches to the wall-supports much in the same way that drywall is attached. The customer selects the color combination of their choice. Avonite® can have backlighting creating a safe and warm atmosphere. Avonite® is practically indestructible, never needs painting, is nonporous and seamless, is easily cleaned, and is built off-site to exact dimensions.

### Overhead Surgical Lighting

The traditional overhead operating room light fixtures are removed and replaced with state-of-the-art directional stage-type lighting to both improve the lighting capability in the operating field and free up ceiling space for additional equipment. Such general "stage-type" lighting technology is available from Skytron Corporation. In an alternative embodiment, the lighting is as above but recessed into the ceiling. Further, the typical embodiment uses cool high-intensity halogen lights that are strategically located around a focal point, such as the operating table. The lights are computer controlled and coordinated such that an RF location device directs the lighting to either of several focal spots on the patient. Shadow canceling technology allows for maximum lighting even when the operative field is crowded with personnel.

### Floor Design

The floor is reconfigured to enclose all wiring, cables, electrical equipment and so on. This results in the new floor being essentially flat and completely clear of all obstructions apart from the essential equipment such as the operating room table, anesthesia machine, and tables for instruments and essential supplies. This opens up space for both clear and accident-free circulation around the patient, as well as facilitating floor and room cleaning to decrease room turnover time. Further, corners, including those formed by the walls and floor, are rounded to aid in cleaning.

### Ultraviolet Sterilization

Each suite is equipped with ultraviolet light and ozone room sterilization. This ensures that all floors, walls, and surfaces are clean. The sterilization devices will be incorporated into the ceiling. In one embodiment, the sterilizer can be automated to sterilize after a computer or CPU has determined the room is empty. This process can also be done at the end of each shift. The operating room incorporates airtight doors and fixtures to allow fumigation with ozone without exposure to personnel outside the operating room.

In addition, the operating room will include sink-trap sterilizers for any sinks that might be incorporated therein. The sterilizers will irradiate pooled-water and surfaces in sinks thereby eliminating highly resistant pathogens and preventing their release into the surgical environment.

### Floor Cleaning

With the floor clear of substantial equipment and obstructions, an automated robotic cleaner may be used thereby speeding up room cleaning and turnover time. Robotic floor cleaners (e.g., Floor Genie™) are redesigned to work in a surgical environment where sterility, rather than mere cleanliness is the goal. The robotic-floor cleaner and sterilization system cleans floors between cases. The system has disposable cleaning cassettes ensuring a sterile environment. The Floor Genie™ robotic floor cleaner reduces the "turnover time" required between cases, as it operates simultaneously while the staff prepares the room for the next case. The rounded corners, as described above, may be adapted to facilitate the robot device's cleaning, as by appropriately rounded corners sufficient to allow access to the robot device.

### Waste Disposal

Trash receptacles are strategically placed throughout the room. For example, two sets of three receptacles are located conveniently throughout the room. Each receptacle is designated for sharp objects, biologics, and garbage respectively. The receptacles are made of lightweight, disposable materials and are insertable into O-rings in the walls. The receptacles can also receive waste bags or, as for sharps, specialized cartridges. Typically, only the bags need be replaced when the receptacle is full. If there is accidental breakage of a bag, the receptacles can be removed and a new set inserted. The receptacles or O-ring snaps (used to hold the waste bags into place by snapping over said bags and onto the cartridges) may also be color-coded for quick identification of the type of waste to be inserted. In an alternative embodiment, the receptacles may be on the wall mounted while still using a replaceable cartridge and bag design, such as for when structural limitations prevent in-wall mounting.

### Holistic Effect

This operating room uses a series of new, innovative technologies that cumulatively and synergistically overcome many current problems and issues. It creates an attractive alternative to existing operating room environments for the specific purpose of attracting new surgeons, thereby providing incremental surgical volume. It also creates a new, state-of-the-art environment for surgical and specialized diagnostic testing facilities, not just a modification of the current obsolete design.

The features of the operating room can be implemented for different applications, including surgery, cardiac catheterization laboratories, ambulatory surgical facilities, and diagnostic special procedures suites. The remodeling of the operating room is applicable to many surgical specialties and reflects the requirements of many different surgeons and health care professionals; it is not limited as solely a surgical suite. Finally, the operating room design provides hospital staff with safety, simplicity, integration, and a sense of calm; for the patient, it provides safety, a sense of calm, and a new degree of confidence.

### BRIEF DESCRIPTION OF THE DRAWINGS

So that the manner in which the above-recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments.
- FIG. 1 is a top plan view showing a floor plan of a hospital operating room in accordance with the invention;
- FIG. 2 is a perspective view of an interior wall of the operating room comprising "post-office" style compartments with doors in accordance with the invention;
- FIG. 3 is a side elevation of an interior wall of the operating room comprising waste-receptacles and a monitor in accordance with the invention;
- FIG. 4 is a perspective view of two interlocking floor support columns;
- FIG. 5 is a top plan view of an underfloor layout of the operating room in accordance with the invention;
- FIG. 6 is a side cross-sectional view of a pod in the raised position incorporated into standard flooring;
- FIG. 7 is a side cross-sectional view of the pod depicted in FIG. 6 in a lowered position incorporated into standard flooring;
- FIG. 8 is a perspective view of one embodiment of the pod in its retracted state.
- FIG. 9 is a side-view of the pod and a surgical cart, said cart docking with said pod.
- FIG. 10 is a side cross-sectional view of a pod in the raised position incorporated into raised flooring;
- FIG. 11 is a side cross-sectional view of the pod depicted in FIG. 10 in a lowered position incorporated into raised flooring;
- FIG. 12 is a side view detail of docking operation between the pod top surface and bottom of an anesthesiology cart;
- FIG. 13 is a perspective view of the pod showing a docking compartment and a convenience outlet on the side;
- FIG. 14 is a side elevation view of a patient table in accordance with the invention;
- FIG. 15 is a side elevation view of a C-arm imaging device in accordance with the invention;
- FIG. 16 is a side elevation view of the imaging device (retracted) depicted in FIG. 15;
- FIG. 17 is a side elevation view in partial cutaway of a robotic floor cleaner in accordance with the invention;
- FIG. 18 is a top plan view of the disposable portion of the robotic floor cleaner depicted in FIG. 17;
- FIG. 19 is another embodiment of the surgical table shown in a side elevation view.

### DETAILED DESCRIPTION OF THE DRAWINGS

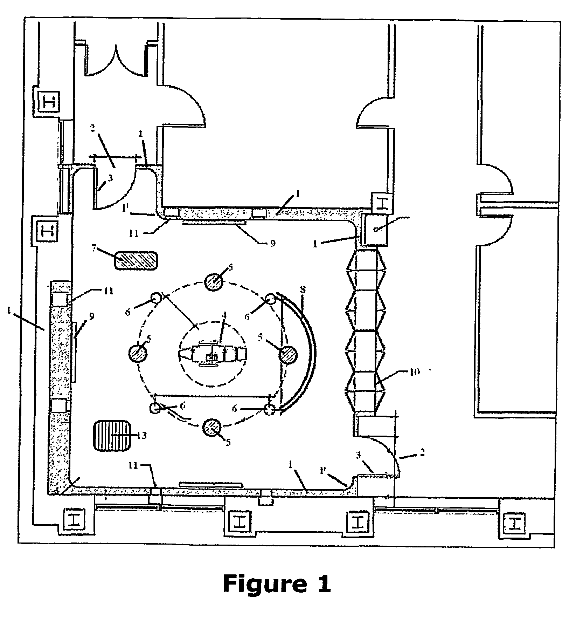

As shown in the drawing FIGS. 1-19, a surgery operating room in a medical facility having no wires, high-tension cables, or hoses exposed to the working environment within the operating room is presented. The operating room includes one or more of the following in its various embodiments:
1. walls with rounded corners and a ceiling, at least one of said walls comprising built-in compartments for supplies, said compartments adapted to be accessible from inside and outside said room, and at least one of said walls having in-wall receptacles for disposing of biologics, sharps, and trash;
2. a sub-floor and a finished floor optionally supported by interlocking floor support columns with each column dovetailed with adjacent columns; the finished floor may optionally be raised to provide a sub-flooring supported by concrete or other materials.
3. at least one pod with utility connections, in which said utility connections are for supplying utilities and data, said pod or pods mounted between said sub-floor and said finished floor, and movable between a lowered position in which an upper surface of said pod is flush with said finished floor and an upper position in which said pod is raised above said finished floor;
4. a surgical table mounted on the sub-flooring of said operating room, said surgical table being powered to raise, lower, and/or orient said surgical table as required;
5. a robotic floor cleaner with sterile, disposable cleaning cartridges;
6. translucent backlit panels disposed on the interior walls;
7. a ceiling-mounted imaging C-arm movable between a parked position against said ceiling and positions oriented to obtain imaging of a patient;
8. flat-panel monitors disposed in or on one or more of said walls for real-time displays;
9. a ceiling-mounted surgical light or array thereof adapted to provide shadow cancellation and automatic focus following of and by placement of a wireless RF focus locator;
10. wireless controls for control over apparatuses in said operating room;
11. at least one ceiling-mounted UV and ozone room sterilization device;
12. at least one UV sink-trap sterilization device for any sinks optionally comprised in the present invention;
13. a multiple frequency USB to radio frequency (RF) to USB paired input and output communication system wirelessly connecting surgical equipment, diagnostic imaging equipment, anesthesia monitoring, navigational devices and surgical instruments to at least one wall-mounted high definition display monitor for imaging and patient information visualization in real time;
14. other design changes, methods, and devices as described below.

### Generally

The surgery operating room has walls with rounded corners and a ceiling, wherein one of the walls optionally has an array of built-in compartments for carrying medical supplies. These compartments are accessed from inside the operating room to retrieve supplies as needed and are accessed from outside of the room for refilling and re-stocking the compartments with medical and surgical supplies. The operating room is configured to provide no sharp corners, which are difficult to clean. All walls intersect in rounded corners having a radius sufficient to allow cleaning by human or specially adapted robot or other device.

The surgery operating room optionally also includes a ceiling mounted imaging C-arm movable between a parked position against the ceiling and lowered positions oriented to obtain imaging of a surgical patient.

Additionally, the surgery operating room also preferably has flat-panel monitors embedded in or on one or more walls of the operating room for real-time displays of patient and utility supply information.

Optionally, a cabinet extends along a wall of the operating room for housing compartments containing receptacles for disposing of wastes, such as biologics, sharps and trash. Alternatively and preferably, receptacles are placed in-wall for receiving of said wastes.

The surgery operating room may have ceiling mounted theater-type lights with (preferably) or without shadow cancellation technology and the ability to focus and follow a point by placement of a wireless RF focus locator. Wireless controls control, among other things, movement of the table, C-arm, pods, and docking a cart to a pod.

Robotic cleaning of the finished floor of the operating room is optionally provided by a battery operated robotic floor cleaner with sterile disposable elements. The cleaner and disposable elements are adapted for rapid cleaning and sterilizing of the finished floor after an operation or other medical procedure to make the operating room ready for a subsequent operation.

The finished floor may be supported by interlocking floor support columns with each column dovetailed with adjacent columns, thereby providing an extremely rigid floor with outstanding weight-bearing ability. Alternatively, the existing or standard operating room floor may be used. Either floor type is adapted to provide a watertight seal that complies with hospital operating room requirements. For example, the pods are sealed about their perimeter with water-resilient O-rings that would withstand dousing with water for an extended period, such as 20 minutes.

The operating room of this invention also preferably has no wires, tubes, high-tension cables, or hoses exposed to the working environment. All wall coverings in the operating room are adapted to be washed down repeatedly without deterioration. Also, ultraviolet light and ozone room sterilization will be installed so that all surfaces can be sterilized at the end of each shift. Further, ultraviolet sterilization will be utilized to sanitize sink-traps in any sinks disposed within the operation room. Sterilization is facilitated by the presence of doors that seal airtight, thereby allowing fumigation. The walls, ceiling, and floor are also designed to be resistant to gas leakage.

### FIGS. 1-17.

The floor layout of FIG. 1 shows the perimeter **1** with entrances **2** and airtight doors **3** (fewer or more doors and entrances are contemplated). A wall of post-office style storage cabinets **10** is shown. The perimeter is adapted to provide rounded corners **1'** with a radius sufficient to allow easy cleaning by human, robot, or cleaning device. A typical radius is between 3 to 5 inches. The doors are adapted to be airtight such as with pneumatic or compression-gasket sealing technologies. The cabinets **10** are in communication with the hallway for remote inventory control, although any geographic location communicating with a hallway is applicable. Any device that communicates with areas outside the operating room is adapted with pneumatic, compression-gasket sealing, or other technologies to provide a room substantially airtight. In a preferred embodiment, the room would be sealable to allow fumigation as required in a Biosafety Level 4 Laboratory (BSL 4).

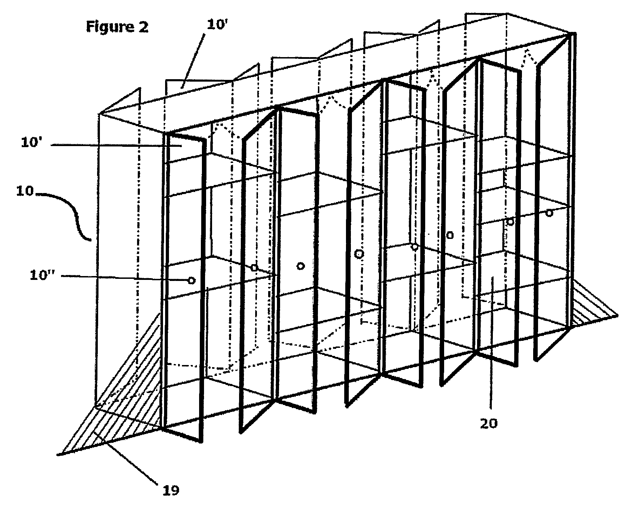

FIG. 2 shows a perspective view of the "post-office" wall **10** with individual compartments, such as **20**, of different sizes. Interior walls **19** are covered with translucent backlit panels of acrylic or polyester resins such as the commercially available Avonite^{™}. Also shown are doors **10**', glass in one embodiment, which close to provide an airtight seal, and door handles **10**".

Continuing with FIG. 1, a surgical table **4** is centrally located on a fixed base. It is accessible to a preferably ceiling mounted C-arm imaging device **8** and surrounded by ceiling mounted theater type lights **5** that have shadow cancellation computer control and automatic focus following by placement of a wireless RF focus locator. Any number of lights maybe disposed about the table **4**; in a preferred embodiment, 8 lights are disposed about the table, equidistant from each other and in a circle. Some of the high-definition monitors 9 are shown on the walls. Laser pointers can be used to interact with the displayed information. Cabinets and counter-tops are not shown and in a preferred embodiment are not found in the OR to help save space. Groups of receptacles **11** (usually three per group) for refuse are shown disposed in the walls; one receptacle per group is used for biologics, sharps, or trash. Four floor pods **6** (other numbers of pods are contemplated) are shown around surgical table **4.** Each can interface and dock with anesthesiology cart **7** or any other cart. Pods **6** recede flush with the floor when not in use. Besides providing gasses, vacuum and electrical lines to cart **7**, one or more can be raised and used as a powered base for other medical equipment such as a microscope. A robotic floor cleaner such as a Floor Genie™ **13** is also shown. The optional ultraviolet light and ozone room sanitizer may be placed at any appropriate point along the ceiling, although in a preferred embodiment it is centrally located to allow maximal dispersion of ultraviolet light rays.

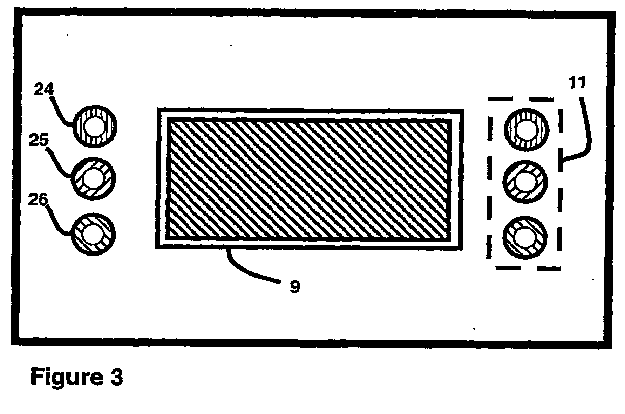

FIG. 3 shows a wall with one embodiment of the receptacles **24**, **25** and **26** forming arrays **11** for biologics, sharps, and trash respectively. In this embodiment, the receptacles are arrayed vertically. High-definition monitor **9** is also shown.

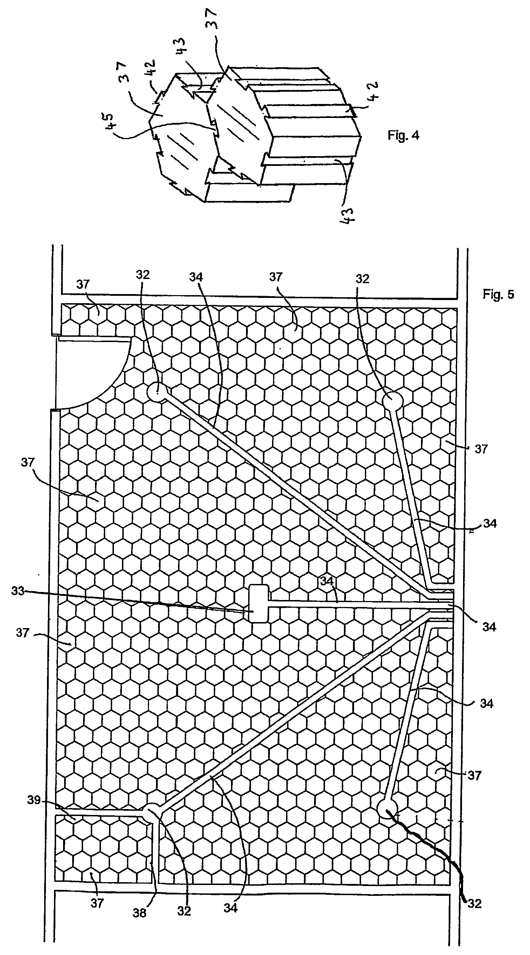

A close-up view of two support columns **37**, which are used in one embodiment of the invention (particularly for use with a raised floor) is shown in FIG. 4, where each column **37** has a dovetail feature in the center of each flat wall. Alternate sides have either protruding features **42** or recessed features **43** that can interlock as shown at **45** by sliding one column into an adjacent one. The top surfaces are shown solid and co-planar when supported by a flat concrete subfloor. However, top surfaces are not required since a structural panel layer can be laid on top of short (approximately 10" high) columns **37.** In an alternative embodiment, if columns **37** do not have top surfaces, they can be manufactured by extrusion (in addition to molding). This may be more economical. A fiber-reinforced resin can also be used. After columns **37** are installed in all the spaces adjacent to pod housings **32** and **33** and conduit paths **34**, sheet flooring is laid down. Then a top floor surface is poured on top for a seamless, easy to clean surface.

FIG. 5 shows an embodiment of the operating room having a specially designed floor using support columns **37.** This floor is preferably used when a raised finished floor is incorporated into the operating room. In one preferred embodiment, the operating room floor is not raised. Therefore, it does not incorporate interlocking support columns **37.** The pod housing conduits, housings, and the like underfloor assemblies may nonetheless be disposed of as described in FIG. 5 in the standard operating room floor. Pod housings **32** such as for a surgical cart or an anesthesia cart **7** (not shown) and pod housings **33** for surgical table **4** (not shown) are shown. The pods need not be docked with a cart but may also be used as normal utility sources by direct connection with a device. While pod housings **32** are shown in FIG. 5 as being circular in shape, they may have any other suitable geometric shape, such as square, ovoid, rectangular, triangular and other polygon shapes. Electrical/gas conduit paths **34** to these pods are also shown emerging past one wall of the operating room.

The interlocking hexagonal floor support columns **37** can optionally define a conduit path on their corners **39** or along flat sides **38.** These hexagonal floor support columns **37** are shown throughout the floor of the operating room. Although other shapes, such as a square, or other geometric shapes can be used, the hexagonal honeycomb structure is preferred because it is extremely rigid and has outstanding weight-bearing capability.

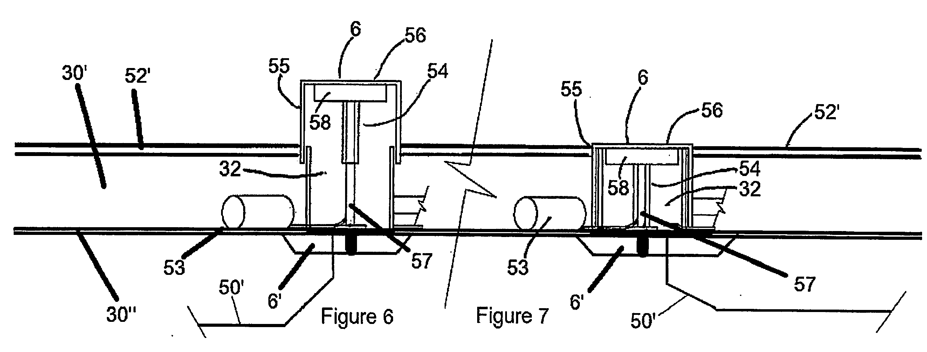

The flooring shown in FIG. 5, which incorporates the dovetail interlocking support columns **37**, are used when a raised finished floor is desired. As stated, in an alternative and preferred embodiment, the floor pod housings **32** are laid out as shown in FIG. 5 but do not incorporate columns **37**. Instead, the floor pods are inserted into the concrete sub-floor already existing, by, for example, wet cutting the floor slab and anchoring the bottom of the floor-pod below the concrete slab. FIGS. 6, 7, 10, and 11 more clearly indicate the two, of other, possible floor arrangements.

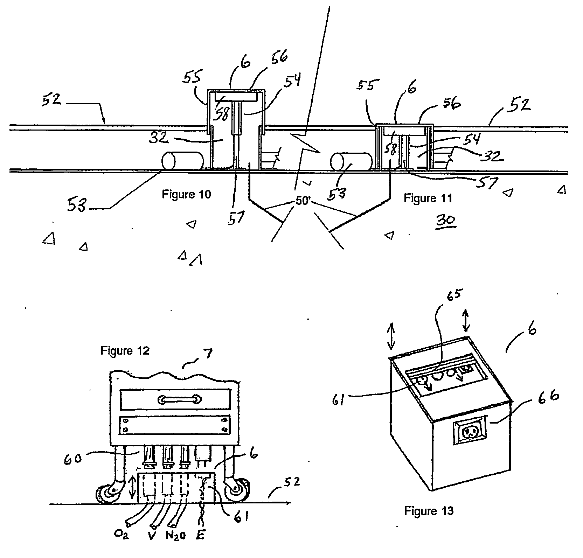

Pods **6** are shown in cross-section in housings **32** in FIG. 6 (in a raised position) and FIG. 7 (in a lowered position). The pods are placed into cavities formed by, for example, wet-cutting areas for the pods and are anchored below the floor material **30**' at **30**" by floor pod anchor **6**' (as opposed to the alternative arrangement shown in FIGS. 10 and 11 in which the pods are placed inside a raised floor). A piston **57** within cylinder **54** and support block **58** are used for raising or lowering pod **6** via lifting forces, such as by fluid or other means from either an electro-pneumatic, electro-hydraulic, or (preferably) electro-mechanical generator **53.** Pods **6** have side housings **55** that are attached. Any side of pod 6 such as side housing **55** or the top surface **52** may be adapted to provide connections to utilities or means for docking with devices such as a specially designed surgical cart, thereby providing utilities to these or other devices. The top surface **52** of the finished floor will be flush with top of pod **6** when lowered as seen in FIG. 7. All wires **50**', cables **50**', and hoses **50**' may be run through a channel in the subfloor **30'** or beneath the subfloor **30**".

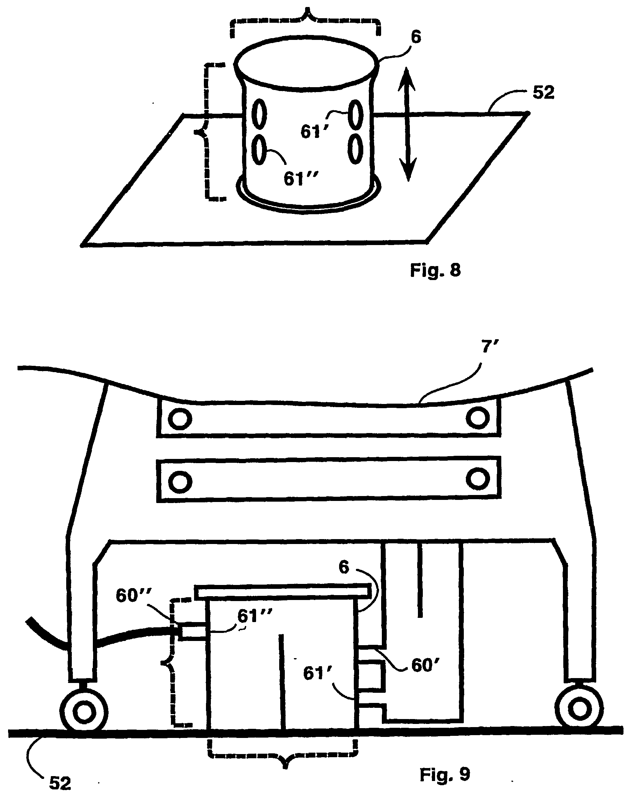

FIG. 8 shows a view of the pod described in FIG. 9 undocked and unconnected. Receptacles **61**' and **61**" are shown as well as floor **52**. The top edge of pod **6** also seals against the floor **52**, thereby preventing water drainage down the sides of the device.

FIG. 9 shows a pod docked with a surgical cart. The relationship between the first set of male connectors **60**' on the side docking plate of cart **7'** and the female receptacles **61'** within the side of pod **6** during docking is shown. Receptors **61'** are specially designed for connection with a surgical cart adapted for docking. The relationship between the second set of female connectors **61"** and an outside connection **60",** such as to a device requiring electricity, is shown. The receptacles **61"** are designed to be adapted to a variety of utilities. The connections may be reversed in an alternative embodiment in which, for example, male connectors **60'** are female receptacles and female receptacles **61'** are male connectors. Under floor **52,** female receptacles **61'** and **61"** are connected to various utilities. In this embodiment, it is preferred that the male and female receptacles providing gases are of a universal type, i.e. a single adaptor will be used for all gas, scavenging, aspirating, and vacuum connections.

Pods **6** (in an alternative embodiment) are shown in cross-section in housings **32** in FIG. 10 (in a raised position) and FIG. 11 (in a lowered position). Concrete subfloor **30** is shown below a raised floor **52** (optionally supported by support columns **37** (not shown)). A piston **57** within cylinder **54** and support block **58** are used for raising or lowering pod **6** via lifting forces, such as by fluid or other means from either an electro-pneumatic, electro-hydraulic, or (preferably) electro-mechanical generator **53.** Pods **6** have side housings **55** that are attached. Any side of pod **6** such as side housing **55** or the top surface **56** may be adapted to provide connections to utilities or means for docking with devices such as a specially designed surgical cart, thereby providing utilities to these or other devices. The top surface **52** of the finished floor will be flush with top of pod **6** when lowered as seen in FIG. 8. All wires **50',** cables **50',** and hoses **50'** may be run through a channel in the subfloor **30** or beneath the raised floor and the optional support columns **37** (not shown).

FIG. 12 shows another possible embodiment of the pod. The relationship between the male connectors **60** on the bottom docking plate of cart **7** and the female receptacles **61** within the top end of pod **6** during docking is shown. The connections may be reversed in an alternative embodiment in which male connectors **60** are female receptacles and female receptacles **61** are male connectors. Under floor **52**, female receptacles **61** are connected via hoses to oxygen, vacuum, nitrous oxide, and via cable to provide electrical power. In an alternate embodiment, the male and female receptacles for gasses may be of a universal type, i.e. a single adaptor will be used for all gas, scavenging, aspirating, and vacuum connections.

FIG. 13 shows a close-up of the top surface of one example of pod **6**, showing a sealable compartment where female receptacles **61** are exposed by sliding of automatic door **65.** When door **65** is closed, it seals watertight so that the top surface can be washed down. The top edge of pod **6** also seals against the floor **52**, thereby preventing water drainage down the sides of the device. Hospital-grade convenience outlet **66** is also provided for equipment that may be placed on raised pod **6** (besides anesthesiology cart **7**).

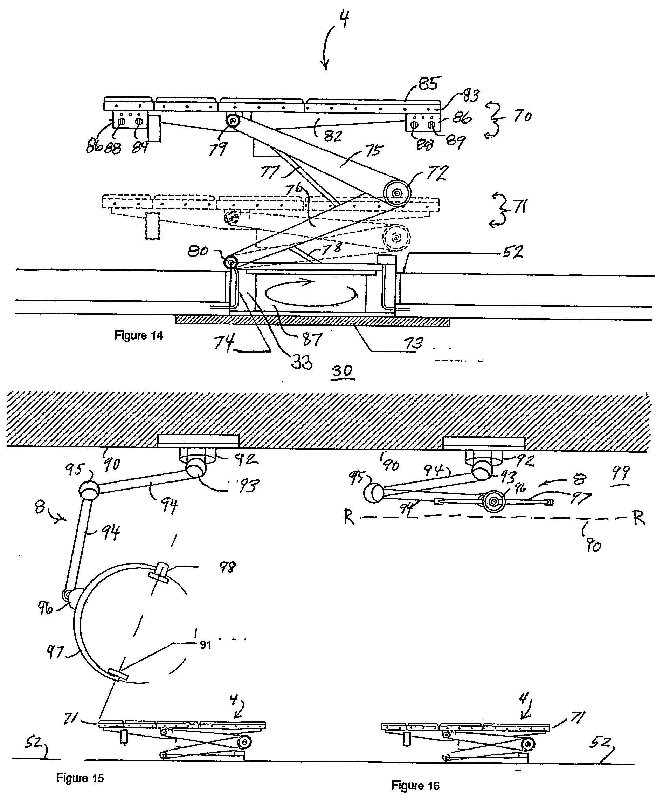

FIG. 14 is a side view of one embodiment of the surgical table **4** in raised position **70** or in lowered position **71** above floor **52** of the operating room. Pod housing **33** is provided for surgical table **4.** Surgical bed **4** is solidly attached to a strong sub-base, such as, for example, steel plate **73**, which is bolted or otherwise attached into the concrete sub floor **30.** Powered braces **77** and **78** are used to raise and lower top frame **83** with pads **85** via arms **75** and 76 riding in pivots **72**, **79**, and **80.** Robustly built to support heavy patients, such as, for example, a 550-pound patient, table **4** has upper support rib **82**; and the tabletop of table **4** can cantilever, rotate, and slide. All utilities, such as power, vacuum, electrical, and gas, are fed through pod housing **33** or other below floor space if a pod housing is not provided. As illustrated by lines **74** (which comprise wires, cables, and hoses) said lines run beneath the floor. These lines are carried to outlet/inlet **86** at either head or toe ends. If necessary, utility outlet/inlets **86** have utility connections **88** and **89** and are accessible at either end of table **4.** FIG. 14 also shows that surgical table **4** can be rotated 360 degrees about rotation base **87** within pod housing **33.**

The ceiling mounted imaging member, such as C-arm **8**, is illustrated in its lowered operating position in FIG. 15 and in its raised "parked" position at the ceiling **90** in FIG. 16. Through recent advances in technology relating to the use of photomultipliers **91** which require very low intensity X-rays via head **98**, these devices have been miniaturized and lightened to a great extent. This is what makes ceiling attachment **92** a practical option. C-arm **97** may be titanium alloy or carbon fiber. Arms **94**, riding in joints **93**, **95** and **96**, such as ball joints or air bearings, form a true six degree-of-freedom robot with very smooth operation at reasonably low cost. While imaging C-arm **8** is shown adjacent to ceiling **90**, it can also retract optionally into a recess (not shown) within ceiling **90**, so that it is flush with, or recessed within, an optional ceiling recess **99** of ceiling **90**, wherein the bottom of ceiling recess **99** is indicated by dashed line "R-R" in FIG. 16.

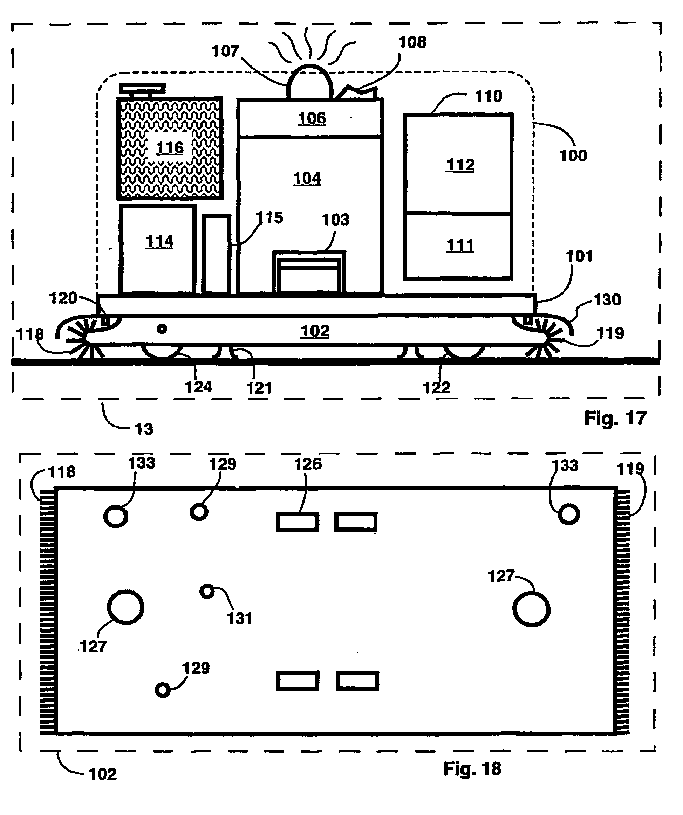

FIGS. 17 and 18 shows a modified robotic floor cleaner, such as a modified Floor Genie™. The floor cleaner incorporates sterile disposable elements. FIG. 14 shows Floor Genie™ **13** with cover **100** shown in a cutaway view to reveal its interior, and to show the placement of some of the major components. Reference numeral **101** is the chassis of the reusable portion of Floor Genie™ **13.** Portion **102** below is a disposable unit that is resupplied in a sterile pack, with connections to reusable chassis portion **101**. An optional bumper **130** may be provided around Floor Genie™ **13**. Disposable portion **102** of Floor Genie™ **13** has wet scrubbing brushes **118** at the front and brushes **119** at the rear. These are connected to, and driven by, motor **115** within the non-disposable, reusable portion **101.** Cleaning fluid in reservoir **116** is sprayed through nozzles **120**, which have back-flow preventers to prevent reverse contamination of fluid supply reservoir **116.** Vacuum cleaner **110** is also provided with motor/impeller **111** and receptacle **112** has vacuum inlets **121** at front and back of disposable portion **102.**

The entire Floor Genie™ cleaner **13** is powered by rechargeable battery pack **104** and is controlled by computer **106.** Flashing light **107** indicates operation. On/Off switch **108** is preferably provided at the top of reusable portion **101.** The drive configuration is similar to that of a zero turning radius riding lawnmower. Here, the two fixed drive wheels **124** are driven by two independent motors **114** near the front. Two passive swiveling casters **122** are near the rear. Side handles **103** with latch bar control coupling and de-coupling from disposable platform **102** that carries both drive wheels **124**, brushes **118** and **119** as well as casters **122.**

FIG. 18 is a top plan view of disposable platform **102** of Floor Genie **13** showing alignment and latching slots **126** that engage with the top reusable portion **101**. Vacuum connections **127** and water connections **133** are illustrated as well as drive motor shaft sockets **129** and brush motor drive socket **131.** Although autonomous and very maneuverable, the accuracy and/or simplicity of the guidance system can be enhanced with waypoint emitters embedded in the floor surface that are detectable by computer **106** via appropriate sensors.

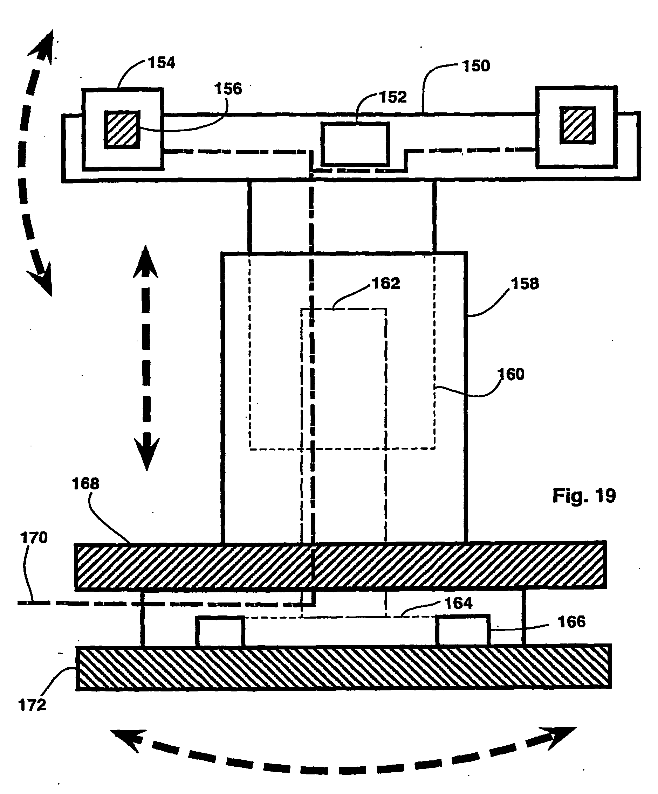

FIG. 19 shows an alternative embodiment of the surgical table **4** shown in FIG. 14. In this conception of table **4**, patient table support means **150** is hinged at point **152**, thereby allowing tilting of the table. Utility box **154** (which can be disposed alone or with other boxes anywhere on table **150**) provides receptacles/connections **156.** All utility lines, connections, wires, cables **170** are fed to the box **154** from within support means **150**, support columns **158** and **160**, and underneath floor **168.** The table is vertically displaceable by movement of column **160** past **158** by an actuator/piston combination **162.** The support column rests on, by support from **162**, bushing/turntable **164** mounted within block **166** thereby allowing rotation. The entire table and column combination is stably anchored by anchor **172**, which is affixed to materials beneath floor **168.** Patient table support means **150** may be removed at the hinge point or additionally at other points of joining to the support column, thereby allowing like patient table support means to be reattached, which are specially configured for particular surgical procedures. The patient table support means share the feature of having utility boxes **154** with internal sourcing of utilities.

In the foregoing description, certain terms and visual depictions are used to illustrate the preferred embodiment. However, no unnecessary limitations are to be construed by the terms used or illustrations depicted, beyond what is shown in the prior art, since the terms and illustrations are exemplary only, and are not meant to limit the scope of the present invention. Designations of a wall geographically are for ease of reference only and do not limit the disposition of a wall and its elements to a particular compass direction.

It is further known that other modifications may be made to the present invention, without departing the scope of the invention, as defined by the appended claims.

## Claims

1. An operating room (OR) comprising:
(a) walls (10, 19) and a ceiling (90);
(b) a sub-floor (30, 30') and a finished floor (52);
(c) a pod system for providing utilities is provided, wherein said pod system comprises at least one pod (6) with utility connections (88, 89')providing utilities, there being at least one said pod (6) mounted between said sub-floor (30, 30') and said finished floor (52); **characterized in** said pod (6) being movable between a lowered position in which an upper surface of said pod is flush with said finished floor (52), thereby freeing any floor space occupied by the pod (6), and an upper position in which said pod (6) is raised above said finished floor (52); said pod system being thereby adapted to occupy floor space only when a utility connection (88, 89) is required.

2. The operating room of claim 1 further comprising at least one surgical light (5).

3. The operating room of claim 1 further comprising a surgical table (4) fixedly mounted on the sub-flooring of said operating room, the table (4) having table support means (150) which share the feature of having utility boxes (154) with internal sourcing of utilities

4. The operating room of claim 1 in which at least one of said walls (10, 19) comprises built-in compartments (20) for supplies, said compartments adapted to be accessible from the inside and outside of said room.

5. The operating room of claim 1 further comprising at least one flat-panel monitor (9) disposed on one or more of said walls (10, 19), there being a communication system adapted to wirelessly connect operating room apparatuses to the at least one flat panel monitor (9).

6. The operating room of claim 1 further comprising in-wall or on-wall receptacles (26) for disposing of waste.

7. The operating room of claim 1 in which said pods are adapted for docking with carts (7) or other apparatuses within said operating room.

8. The operating room of claim 1 further comprising a robotic cleaner (13) in which said robotic cleaner (13) has a sterile, disposable cleaning cartridge and which said robotic cleaner (13) and said cartridge are adapted to provide cleaning and sterilizing of said finished floor (52).

9. The operating room of claim 1 wherein at least one wall (10, 19) comprises translucent backlit panels attached thereto.

10. The operating room of claim 9 in which said translucent backlit panels are made of low dielectric constant materials, non-porous materials, or low dielectric constant and non-porous materials.

11. The operating room of claim 1 further comprising a communication system adapted to wirelessly connect operating room apparatuses to at least one flat-panel monitor (9).

12. The operating room of claim 1 further comprising at least one ceiling-mounted UV, ozone, or UV and ozone sterilization device.

13. The operating room of claim 12 in which said ceiling-mounted UV sterilization device or devices are automated to clean when operating room personnel have left said operating room.

14. The operating room of claim 1 further comprising sink-trap UV sterilizers.

15. The operating room of claim 1 in which at least one corner defined by the intersection of any of said walls (10, 19), of said finished floor (52) and any of said walls (10,19), of said ceiling (90) )and any of said walls (10,19) are rounded.

16. The operating room of claim 1 in which the operating room is capable of being fumigated.

17. The operating room of claim 1 in which said pod (6) is anchored below said sub-floor (30, 30').

18. A method of using an operating room to maximize space and/or increase safety comprising the steps of providing the operating room according to either claim 1 or claim 4.

19. The method of claim 18 further comprising controlling inventory with radio frequency (RF) control by:
a) tagging items with radio-frequency identification (RFID) tags;
b) placing said items on a shelf in said operating room, said shelf having an RFID detector; and
c) having said RFID detector communicate with a computer database of an inventory of said items that said item has been placed on said shelf, said database then indicating to a user said item has been placed on said shelf.

20. The method of claim 19 in which said items are packaged in sterile packaging and said RFID tags are broken when said sterile packaging is opened.

## Patentansprüche

1. Operationsraum (OR), umfassend:
(a) Wände (10,19) und eine Decke (90);
(b) ein Unterboden (30,30') und ein endbehandelter Boden (52);
(c) ein Installationszellensystem zur Bereitstellung von Versorgungeinrichtungen, wobei das Installationszellensystem mindestens eine Installationszelle (6) mit Versorgungsanschlüssen (88,89) zum Bereitstellen von Versorgungseinrichtungen umfasst, wobei es mindestens eine der genannten Installationszellen (6) gibt, die zwischen dem Unterboden (30,30') und dem endbehandelten Boden (52) angelegt ist, **dadurch gekennzeichnet, dass** die Installationszelle (6) zwischen einer abgesenkten Position, in der eine Oberseite der Installationszelle mit dem endbehandelten Boden (52) auf gleicher Höhe ist, wodurch ein von der Installationszelle (6) beanspruchter Platz auf dem Boden frei wird, und einer oberen Position beweglich ist, in der die Installationszelle (6) über den endbehandelten Boden (52) angehoben ist; wobei das Installationszellensystem dadurch derart ausgelegt ist, Platz auf dem Boden nur dann zu beanspruchen, wenn ein Versorgungsanschluss (88,89) benötigt wird.

2. Operationsraum nach Anspruch 1, ferner umfassend mindestens eine Operationslampe (5).

3. Operationsraum nach Anspruch 1, ferner umfassend einen Operationstisch (4), der auf dem Unterboden des Operationsraums unbeweglich aufgebaut ist, wobei der Tisch (4) Mittel zur Aufnahme des Tischs (150) hat, die das Merkmal gemeinsam haben, dass sie über Versorgungskästen (154) mit inneren Zuführungen von Versorgungseinrichtungen verfügen.

4. Operationsraum nach Anspruch 1, wobei mindestens eine der Seitenwände (10,19) eingebaute Fächer (20) für Zuführungen aufweist, wobei die Fächer so ausgebildet sind, dass sie von innerhalb und von außerhalb des Operationsraum zugänglich sind.

5. Operationsraum nach Anspruch 1, ferner umfassend mindestens einen Flachbildschirm-Monitor (9), der an einer oder mehreren der Seitenwände (10,19) angeordnet ist, wobei es ein Kommunikationssystem gibt, das ausgelegt ist, um kabellos Apparate des Operationsraums an mindestens einem Flachbildschirm-Monitor (9) anzuschließen.

6. Operationsraum nach Anspruch 1, ferner umfassend Wandversenkte oder auf der Wand montierte Aufnahmegefäße (26) zum Wegwerfen von Abfall.

7. Operationsraum nach Anspruch 1, in welchem die Installationszellen (6) zum Andocken von Wagen (7) oder anderen Apparaturen in dem Operationsraum ausgebildet sind.

8. Operationsraum nach Anspruch 1, ferner umfassend einen Reinigungsroboter (13), wobei der Reinigungsroboter (13) eine sterile Einweg-Reinigungspatrone hat und wobei der Reinigungsroboter (13) und die Patrone ausgelegt sind, um das Reinigen und Sterilisieren des endbehandelten Bodens (52) zu gewähren.

9. Operationsraum nach Anspruch 1, wobei mindestens eine Seitenwand (10,19) darauf aufgebrachte, lichtdurchlässige, rückseitig beleuchtete Felder aufweist.

10. Operationsraum nach Anspruch 9, in welchem die lichtdurchlässigen, rückseitig beleuchteten Felder aus Materialien mit niedriger Dielektrizitätskonstante, nichtporösen Materialien oder Materialien mit niedriger Dielektrizitätskonstante und nichtporösen Materialien gefertigt sind.

11. Operationsraum nach Anspruch 1, ferner umfassend ein Kommunikationssystem, das ausgelegt ist, um Apparaturen des Operationsraums kabellos mit mindestens einem Flachbildschirm-Monitor (9) zu verbinden.

12. Operationsraum nach Anspruch 1, ferner umfassend mindestens eine deckenmontierte UV/Ozon- oder UV- und Ozon-Sterilisationsvorrichtung.

13. Operationsraum nach Anspruch 12, in welchem die deckenmontierte UV-Sterilisationsvorrichtung oder Sterilisationsvorrichtungen automatisiert sind um zu reinigen, wenn Personal des Operationsraums den Operationsraum verlassen hat.

14. Operationsraum nach Anspruch 1, ferner umfassend Siphon-UV-Sterilisatoren.

15. Operationsraum nach Anspruch 1, in welchem mindestens eine Ecke, die festgelegt ist durch Schnitt einer beliebigen der Seitenwände (10,19), des endbehandelten Bodens (52) und einer beliebigen der Seitenwände (10,19), der Decke (90) und einer beliebigen der Seitenwände (10,19), gerundet ist.

16. Operationsraum nach Anspruch 1, wobei der Operationsraum begast werden kann.

17. Operationsraum nach Anspruch 1, in welchem die Installationszelle (6) unterhalb des endbehandelten Bodens (30, 30') verankert ist.

18. Verfahren zur Anwendung eines Operationsraums, um den Platz zu maximieren und/oder die Sicherheit zu erhöhen, umfassend die Schritte des Bereitstellens des Operationsraums entweder nach Anspruch 1 oder Anspruch 4.

19. Verfahren nach Anspruch 18, ferner umfassend das Kontrollieren des Inventars mit Hilfe von Hochfrequenz(HF)-Überwachung durch
a) Kennzeichnen von Gegenständen mit Hochfrequenz-Identifikations(RFID)-Tags;
b) diese Gegenstände in dem Operationsraum auf ein Bord stellen, wobei das Bord einen RFID-Detektor hat; und
c) den RFID-Detektor mit einer Computer-Datenbank eines Inventars dieser Gegenstände kommunizieren lassen, die auf das Bord gestellt wurden, wobei die Datenbank dann einem Anwender anzeigt, dass dieser Gegenstand auf das Bord gesetzt worden ist.

20. Verfahren nach Anspruch 19, bei welchem die Gegenstände in steriler Verpackung verpackt sind und diese RFID-Tags aufgebrochen werden, wenn diese sterile Verpackung geöffnet wird.

## Revendications

1. Salle d'opération (OR) comprenant :
(a) des murs (10, 19) et un plafond (90) ;
(b) un sous-plancher (30, 30') et un plancher fini (52) ;
(c) un système de nacelles destiné à fournir des utilitaires, le système de nacelles comprenant au moins une nacelle (6) avec des connexions d'utilitaires (88, 89') fournissant des utilitaires, au moins une nacelle (6) étant montée entre ledit sous-plancher (30, 30') et ledit plancher fini (52) ; **caractérisée en ce que** ladite nacelle (6) est déplaçable entre une position abaissée, dans laquelle une surface supérieure de ladite nacelle est en affleurement avec ledit plancher fini (52), libérant ainsi n'importe quel espace au sol occupé par la nacelle (6), et une position relevée, dans laquelle ladite nacelle (6) est relevée au-dessus dudit plancher fini (52) ; ledit système de nacelles étant par conséquent adapté pour occuper un espace au sol uniquement lorsqu'une connexion d'utilitaire (88, 89') est requise.

2. Salle d'opération selon la revendication 1, comprenant en outre au moins une lumière chirurgicale (5).

3. Salle d'opération selon la revendication 1, comprenant ne outre une table chirurgicale (4) montée fixement sur la sous-plancher de ladite salle d'opération, la table (4) présentant des moyens de support (150) partageant la caractéristique de comporter des boîtes d'utilitaires (154) avec approvisionnement interne d'utilitaires.

4. Salle d'opération selon la revendication 1, dans laquelle au moins l'un desdits murs (10, 19) comprend des compartiments (20) pour des fournitures, lesdits compartiments étant adaptés pour être accessibles depuis l'intérieur et l'extérieur de ladite salle.

5. Salle d'opération selon la revendication 1, comprenant en outre au moins un écran plat (9) disposé sur un ou plusieurs desdits murs (10, 19), avec un système de communication adapté pour relier sans fil des appareils de salle d'opération à l'au moins un écran plat (9).

6. Salle d'opération selon la revendication 1, comprenant en outre des réceptacles (26) montés sur le mur ou intégrés dans le mur, pour l'élimination de déchets.

7. Salle d'opération selon la revendication 1, dans laquelle lesdites nacelles sont adaptées pour s'accoupler avec des chariots (7) ou d'autres dispositifs dans la salle d'opération.

8. Salle d'opération selon la revendication 1, comprenant en outre un robot de nettoyage (13), le robot de nettoyage (13) comportant une cartouche jetable stérile, et ledit robot de nettoyage (13) et ladite cartouche sont adaptés pour assurer le nettoyage et la stérilisation dudit plancher fini (52).

9. Salle d'opération selon la revendication 1, dans laquelle au moins un mur (10, 19) comprend des panneaux rétro-éclairés translucides fixés à celui-ci.

10. Salle d'opération selon la revendication 9, dans laquelle lesdits panneaux rétro-éclairés translucides sont constitués de matériaux constants diélectriques, de matériaux non-poreux, ou de matériaux constants diélectriques et de matériaux non-poreux.

11. Salle d'opération selon la revendication 1, comprenant en outre un système de communication permettant de relier sans fil des appareils de la salle d'opération à au moins un écran plat (9).

12. Salle d'opération selon la revendication 1, comprenant en outre au moins un dispositif de stérilisation aux UV, à l'ozone, ou aux UV et à l'ozone, monté au plafond.

13. Salle d'opération selon la revendication 12, dans laquelle ledit ou lesdits dispositif(s) de stérilisation monté(s) au plafond est/sont automatisés pour nettoyer une fois que le personnel de la salle d'opération a quitté ladite salle d'opération.

14. Salle d'opération selon la revendication 1, comprenant en outre des dispositifs de stérilisation de puisards aux UV.

15. Salle d'opération selon la revendication 1, dans laquelle au moins un coin défini par l'intersection de n'importe lequel desdits murs (10, 19), dudit plancher fini (52) et de n'importe lequel desdits murs (10, 19), dudit plafond (90) et de n'importe lequel desdits murs (10, 19) est arrondi.

16. Salle d'opération selon la revendication 1, la salle d'opération pouvant être fumigée.

17. Salle d'opération selon la revendication 1, dans laquelle ladite nacelle (6) est ancrée en dessous dudit sous-plancher (30, 30').

18. Procédé d'utilisation d'une salle d'opération pour maximiser l'espace et/ou augmenter la sécurité, comprenant les étapes de mise à disposition de la salle d'opération selon la revendication 1 ou la revendication 4.

19. Procédé selon la revendication 18, comprenant en outre un inventaire de contrôle, avec un contrôle par radiofréquence (RF), par :
a) le marquage d'éléments avec des étiquettes d'identification par radiofréquence (RFID) ;
b) la disposition desdits éléments sur une étagère dans ladite salle d'opération, ladite étagère comportant un détecteur RFID ; et
c) la communication entre ledit détecteur RFID et une base de données informatique d'un inventaire desdits éléments, pour indiquer que cet élément a été disposé sur ladite étagère, ladite base de données indiquant ensuite à un utilisateur que ledit élément a été disposé sur ladite étagère.

20. Procédé selon la revendication 19, dans lequel lesdits éléments sont emballés dans des emballages stériles et lesdites étiquettes RFID sont rompus lorsque ledit emballage stérile est ouvert.
